# NEUE EUROPÄISCHE PATENTSCHRIFT

(11) **EP 0 614 333 B2**
(45) Veröffentlichungstag und Bekanntmachung des Hinweises auf die Entscheidung über den Einspruch: **29.11.2000**
(45) Hinweis auf die Patenterteilung: 18.12.1996
(21) Anmeldenummer: 92920541.7
(22) Anmeldetag: 29.09.1992
(51) Int. Cl.: A01N 43/68, C07D 251/70

(54) **VERWENDUNG VON 1,3,5-TRIAZIN-2,4,6-TRIS-ALKYLAMINOCARBONSÄUREDERIVATEN ALS BIOZIDE MITTEL**
USE OF 1,3,5-TRIAZINE-2,4,6-TRIS-ALKYLAMINOCARBOXYLIC ACID DERIVATIVES AS BIOCIDAL AGENTS
UTILISATION DE DERIVES DE L'ACIDE 1,3,5-TRIAZINE-2,4,6-TRIS-ALKYLAMINOCARBOXYLIQUE COMME BIOCIDES

(30) Priorität: 19.11.1991 DE 4138090
(43) Veröffentlichungstag der Anmeldung: 14.09.1994
(73) Patentinhaber: CG-CHEMIE GMBH, W-2000 Hamburg 74 (DE)
(72) Erfinder: LESMANN, Jörg, D-2050 Hamburg 80 (DE); SCHÄFER, Hermann, Georg, D-2000 Hamburg 70 (DE)
(74) Vertreter: Glawe, Delfs, Moll & Partner
(86) Internationale Anmeldenummer: EP9202248
(87) Internationale Veröffentlichungsnummer: WO9309670

(56) Entgegenhaltungen:
- EP-A- 0 046 139
- EP-A- 0 262 086
- GB-A- 2 194 782
- US-A- 4 402 907
- CHEMICAL ABSTRACTS, vol. 60, no. 4, 17. Februar 1964, Columbus, Ohio, US; abstract no. 4145e, H. NESTLER ET AL. 'Preparation of N-(1,3,5-triazinyl)amino acid derivatives.' Spalte 4145 ;
- CHEMICAL ABSTRACTS. REGISTRY HANDBOOK - NUMBER SECTION. 1982, COLUMBUS US Seite 156
- CHEMICAL ABSTRACTS. REGISTRY HANDBOOK - NUMBER SECTION. 1991, COLUMBUS US Seite 3274
- E.O.Bennett, J.Am.Soc.Lubric.Engin., Vol.35(3), 137-144 (1979)
- E.O.Bennett et al, J.Gen.Appl.Microbiol., Vol.25, 63-69, (1979)
- H.Nestler & H.Fürst, J.f.praktische Chemie, 4. Reihe, Band 4, 173-185(1963)

## Beschreibung

Die Erfindung betrifft 1,3,5-Triazin-2,4,6-tris-alkylaminocarbonsäurederivate, ihre Verwendung als biozide bzw. biostatische Mittel in wasserhaltigen Systemen und diese enthaltende Kühlschmierstoffe. Die zugrundeliegenden Triazintricarbonsäuren, d.h. die 2,4,6-Tris(omega'-carboxyalkylamino)-1,3,5-triazine, im folgenden 1,3,5-Triazin-2,4,6-tris-alkylaminocarbonsäuren genannt, sind in J. Prakt. Chemie, 23 (1963), S. 173 bis 185, sowie in der EP-B 0 046 139 beschrieben. Die EP-B 0 046 139 betrifft weiterhin die Verwendung der genannten Triazintricarbonsäuren sowie deren Alkalimetall-, Mono-, Di- oder Triethanolammonium-Salze als Korrosionsinhibitoren in wäßrigen Systemen.

Ein Korrosionsschutzmittel, das ein Imidazolin, eine der genannten Triazintricarbonsäuren und mindestens ein Mono-, Di- oder Trialkanolamin und Wasser enthält, ist in der EP-A 0 262 086 beschrieben. Die Korrosionsinhibitoren gemäß EP-B 0 046 139 und EP-A 0 262 086 werden wäßrigen Systemen, z.B. Kühlflüssigkeiten, Kühlschmierstoffen, Anstrichstoffen oder Reinigern, zugesetzt, die ihrerseits weitere Additive, z.B. Biozide, enthalten. Als Additive wurden bisher halogenhaltige Verbindungen sowie z.B. Borsäure und Umsetzungsprodukte von Borsäure mit Alkanolaminen verwendet, siehe Ullmanns Encyklopädie der technischen Chemie, 4. Auflage, Band 8, Verlag Chemie, Weinheim 1974, S. 653-655. In anderen Fällen wurden Formaldehyd oder Formaldehyd-Derivate als Biozid zugesetzt. Halogenhaltige Verbindungen, Borsäure und Borsäurederivate sowie Formaldehyd und dessen Derivate sind jedoch aus verschiedenen Gründen unerwünscht. Daher besteht ein zunehmender Bedarf an bioziden Mitteln zur Verwendung in wasserhaltigen Systemen, die frei von halogenhaltigen Verbindungen, Formaldehyd, Formaldehyd-Derivaten, Borsäure oder Borsäurederivaten sind.

Es wurde nun gefunden, daß 1,3,5-Triazin-tris-2,4,6-alkylaminocarbonsäurederivate der allgemeinen Formel 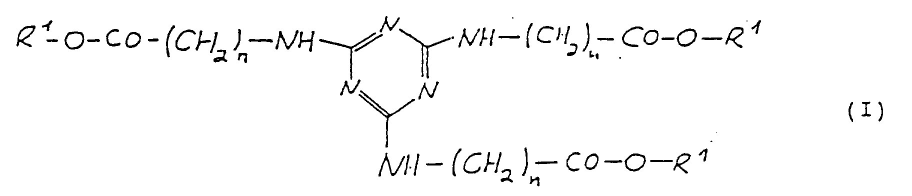 in der
n eine Zahl im Bereich von 4 bis 11 bedeutet und
R¹ eine der folgenden Bedeutungen aufweist:
   a) ein Alkaliatom oder ein Ammoniumion, abgeleitet von einem Alkanolamin der allgemeinen Formel 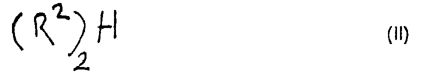 in der
      mindestens eine der Gruppen R²
      aa) eine Hydroxyalkylgruppe mit 2 bis 4 Kohlenstoffatomen,
      bb) eine Hydroxyalkyl-oxyalkylengruppe mit jeweils 2 bis 4 Kohlenstoffatomen in dem Hydroxyalkyl- und Oxyalkylrest, oder
      cc) eine Dihydroxyalkylgruppe mit 3 bis 6 Kohlenstoffatomen
      und, wenn weniger als zwei der Gruppen R² die vorstehende Bedeutung aufweisen, die übrige Gruppe R² eine Alkylgruppe mit 1 bis 6 Kohlenstoffatomen oder Wasserstoff ist,
      bedeutet,
      einen Rest eines Alkanolamins der allgemeinen Formel II
      als biozide bzw. biostatische Mittel in wasserhaltigen Systemen verwendet werden können.

Gemäß einer bevorzugten Ausführungsform der Erfindung werden 1,3,5-Triazin-2,4,6-tris-alkylaminocarbonsäurederivate der allgemeinen Formel I verwendet, bei denen n eine Zahl im Bereich von 4 bis 8, vorzugsweise die Zahl 5, bedeutet.

Alkaliatome können Lithium, Natrium oder Kalium sein.

Die Alkanolamine der allgemeinen Formel II weisen primäre, sekundäre oder tertiäre Amino- und freie Hydroxylgruppen auf. Bei der Umsetzung von primäre oder sekundäre Aminogruppen aufweisenden Alkanolaminen mit Carbonsäuren können sowohl Amide als auch Ester entstehen, die miteinander im Gleichgewicht stehen, siehe "Surfactants in Consumer Products", Hrsg. J. Falbe, Springer-Verlag, Heidelberg 1987, S. 96. Aus Gründen der Übersichtlichkeit sind die Umsetzungsprodukte von 1,3,5-Triazin-2,4,6-tris-alkylaminocarbonsäuren mit Alkanolaminen zu Verbindungen der allgemeinen Formel I, in der R¹ der Rest eines Alkanolamins gemäß der Definition b) ist, hier nur als Aminoester dargestellt. Es ist für den Fachmann aber ohne weiteres ersichtlich, daß unter die so definierten 1,3,5-Triazin-2,4,6-tris-alkylcarbonsäurederivate auch die entsprechenden Alkanolamide fallen.

Typische Beispiele für Hydroxyalkylgruppen mit 2 bis 4 Kohlenstoffatomen, die die Gruppe R² bilden können, sind 2-Hydroxyethyl-, 1-Methyl-2-Hydroxyethyl-, 2-Hydroxypropyl-, 3-Hydroxypropyl-, 2-Hydroxybutyl-, 4-Hydroxybutyl- und 2-Methyl-2-hydroxypropylgruppen; für Hydroxyalkyl-oxyalkylengruppen mit jeweils 2 bis 4 Kohlenstoffatomen in dem Hydroxyalkyl- und Oxyalkylenrest, Hydroxyethyl-oxyethylen-, Hydroxypropyl-oxyethylen-, Hydroxyethyl-diethylenoxy-, Hydroxyethyl-oxypropylen- und Hydroxypropyl-oxypropylengruppen und für Dihydroxyalkylgruppen mit 3 bis 6 Kohlenstoffatomen 2,3-Dihydroxypropyl-, 3,4-Dihydroxybutyl-, 1,3-Dihydroxypropyl- und 1,3-Dihydroxy-2-methyl- oder - ethyl-propylgruppen; weiterhin auch Hydroxyethyl-, Hydroxypropyl- und Hydroxybutyl-oxybutylengruppen.

Verbindungen der allgemeinen Formel I, bei denen R¹ einen Rest eines Alkanolamins der allgemeinen Formel II oder ein von diesem Alkanolamin abgeleitetes Ammoniumion bedeutet, sind durch Umsetzung von 1,3,5-Triazin-2,4,6-tris-alkylaminocarbonsäuren der allgemeinen Formel 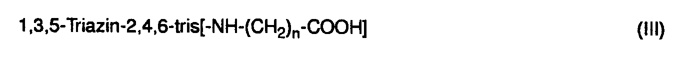 in der n wie oben definiert ist,
mit Alkanolaminen der allgemeinen Formel II nach an sich bekannten Verfahren erhältlich.

Bevorzugt ist die Umsetzung mit einem, bezogen auf die 1,3,5-Triazin-2,4,6-tris-alkylaminocarbonsäuren, molaren Überschuß der Alkanolamine. Der nicht umgesetzte Teil der Alkanolamine kann zur Einstellung eines pH-Wertes von 4,5 bis 9,5 mit organischen Säuren, ausgewählt aus der von geradkettigen oder verzweigten, gesättigten oder ungesättigten Fettsäuren mit 5 bis 22 Kohlenstoffatomen gebildeten Gruppe, umgesetzt werden. Beispiele für die genannten Fettsäuren sind Pentansäure, Hexansäure, Heptansäure, Octansäure, Nonansäure, Decansäure, Undecansäure, Dodecansäure, Tridecansäure, Tetradecansäure, Pentadecansäure, Hexadecansäure, Heptadecansäure, Octadecansäure, Nonadecansäure, Eicosansäure, Heneicosansäure, Docosansäure, 10-Undecensäure, 9c-Dodecensäure, 9c-Tetradecensäure, 9c-Hexadecensäure, 6c-Octadecensäure, 6t-Octadecensäure, 9c-Octadecensäure, 9t-Octadecensäure, 9c,12c-Oc-tadecadiensäure, 9t,12t-Octadecadiensäure, 9c,12c,15c-Octadecatriensäure, 9c,11t,13t-Octadecatriensäure, 9c-Eicosensäure, 5,8,11,14-Eicosatetraensäure, 13c-Docosensäure, 13t-Docosensäure, 4,8,12,15,19-Docosapentaensäure, 12-Hydroxy-Octadecansäure und 12-Hydroxy-9c-octadecensäure, wobei c eine cis-Doppelbindung und t eine trans-Doppelbindung anzeigt, sowie technische Gemische derselben. Besonders geeignet sind weiterhin Fettsäuren bzw. Fettsäuregemische, die aus nachwachsenden Rohstoffen, insbesondere pflanzlichen und/oder tierischen Fetten und Ölen, erhältlich sind, z.B. Capron-, Capryl-, Caprin-, Laurin-, Myristin-, Palmitin-, Stearin-, Öl-, Ricinol-, Linol-, Eruca- und Behensäure.

Bevorzugt wird der nicht umgesetzte Teil der Alkanolamine mit geradkettigen oder verzweigten, gesättigten oder ungesättigten Fettsäuren mit 5 bis 11 Kohlenstoffatomen umgesetzt. Wenn man auf diese Weise keine stabilen Lösungen oder Emulsionen erhält, können zur Einstellung der gewünschten Hydrophil/Hydrophob-Balance zusätzlich geradkettige oder verzweigte, gesättigte oder ungesättigte Fettsäuren mit 12 bis 22 Kohlenstoffatomen verwendet werden.

Bevorzugt werden solche Verbindungen der allgemeinen Formel I eingesetzt, die keine sekundären oder tertiären Aminofunktionen enthalten. Sekundäre Amine bzw. Alkanolamine können mit Nitritionen unerwünschte, stabile Nitrosamine bilden. Tertiäre Amine bzw. Alkanolamine können unter Umständen durch Dealkylierung sekundäre Amine bzw. Alkanolamine bilden. Dagegen bilden primäre Amine in der Regel keine stabilen Nitrosamine, sondern dienen vielmehr wegen des schnellen Zerfalls der intermediär gebildeten Nitrosamine als Abfänger für Nitritionen. Wenn man dennoch von sekundären Alkanolaminen abgeleitete Verbindungen der allgemeinen Formel I einsetzen will, ist die Verwendung eines Gemisches von von primären und sekundären Alkanolaminen abgeleiteten Verbindungen bevorzugt, da dann die Bildung der instabilen primären Nitrosamine schneller erfolgt als die der sekundären Nitrosamine.

Somit betrifft die Erfindung gemäß einem weiteren Aspekt die Verwendung von Verbindungen der allgemeinen Formel I, die frei von sekundären oder tertiären Aminofunktionen sind und somit keine stabilen Nitrosoverbindungen bilden können bzw. die bei gleichzeitiger Anwesenheit der analogen sekundäre oder tertiäre Aminofunktionen enthaltenden Verbindungen der allgemeinen Formel I die Ausbildung stabiler Nitrosoverbindungen verhindern.

Es können auch biozide bzw. biostatische Gemische von Monocarbonsäurealkanolamiden und 1,3,5-Triazin-2,4,6-tris-alkylaminocarbonsäure-alkanolamiden sowie ggf. Alkanolammoniumsalzen der Monocarbonsäuren und/oder der 1,3,5-Triazin-2,4,6-tris-alkylaminocarbonsäuren verwendet werden.

Die vorgenannten bioziden bzw. biostatischen Gemische können durch Vermischung der Einzelkomponenten hergestellt werden. Zweckmäßigerweise werden sie jedoch hergestellt, in dem man die Alkanolamide bzw. Alkanolammoniumsalze in situ aus den Monocarbonsäuren und den 1,3,5-Triazin-tris-alkylaminocarbonsäuren der allgemeinen Formel III, in der n wie oben definiert ist, mit Alkanolaminen der allgemeinen Formel II, in der R² wie oben definiert ist, vorzugsweise in einem Überschuß der Alkanolamine, herstellt.

Vorzugsweise werden primäre Alkanolamine oder Gemische aus primären und sekundären Alkanolaminen verwendet.

Bevorzugt werden pro Mol der 1,3,5-Triazin-2,4,6-tris-alkylaminocarbonsäuren 10 bis 50 Mol, insbesondere 10 bis 30 Mol, der Amine der allgemeinen Formel II und 0,5 bis 5 Mol der Monocarbonsäuren umgesetzt.

Die Umsetzungen werden bei einer Temperatur im Bereich von 50 bis 180°C durchgeführt. Bei Temperaturen von 50 bis 100°C erhält man bevorzugt Alkanolammoniumsalze; bei Temperaturen von > 100 bis 180°C, insbesondere 130 bis 180°C, erhält man Alkanolamide.

Als Monocarbonsäuren werden geradkettige oder verzweigte, gesättigte oder ungesättigten Fettsäuren mit 3 bis 22, insbesondere 12 bis 22 Kohlenstoffatomen, verwendet, die in einer ersten Stufe mit den Alkanolaminen umgesetzt werden, gefolgt von einer Zugabe und Umsetzung der 1,3,5-Triazin-2,4,6-tris-alkylaminocarbonsäuren in einer zweiten Stufe. Diese Umsetzung kann auch in einer anderen Reihenfolge oder in einer einzigen Stufe durchgeführt werden, wobei dann aber weniger ausgeprägte biozide bzw. biostatische Eigenschaften des Gemisches erhalten werden.

Weiterhin werden als Monocarbonsäuren Ethercarbonsäuren der allgemeinen Formel 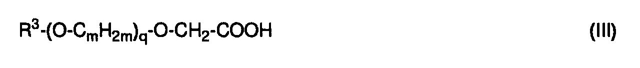 in der
- R³: eine geradkettige oder verzweigte Alkyl- oder Alkenylgruppe mit 9 bis 18 Kohlenstoffatomen,
- m: die Zahl 2 und/oder 3 und
- q: eine Zahl im Bereich von 0 bis 100, vorzugsweise von 0 bis 20, bedeuten,
verwendet werden. Die Umsetzung kann hier in beliebiger Reihenfolge, aber auch in einer einzigen Stufe erfolgen.

Ebenso werden als Monocarbonsäuren Arylsulfonamidocarbonsäuren der allgemeinen Formel 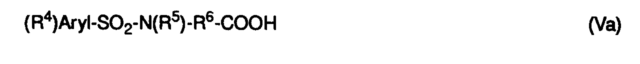 in der
R⁴ Wasserstoff oder eine Methyl- oder Ethylgruppe oder mehrere, R⁵ Wasserstoff, eine Methyl-, Ethyl-, beta-Cyanoethyl- oder Hydroxymethylgruppe, R⁶ eine Alkylengruppe mit 4 bis 6 Kohlenstoffatomen und Aryl einen Phenyl-, Naphthyl- oder Anthracenylrest bedeuten, und/oder Alkylsulfonamidocarbonsäuren der allgemeinen Formel 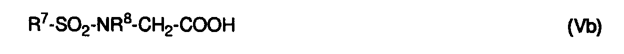 in der R⁷ eine geradkettige oder verzweigte Alkylgruppe mit 12 bis 22 Kohlenstoffatomen und R⁸ Wasserstoff oder die Gruppe -CH₂-COOH bedeuten,
und/oder Halbester bzw. Halbamide der allgemeinen Formel Vc 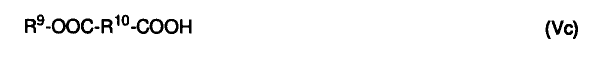 in der
R⁹ der Rest eines Alkanolamins der allgemeinen Formel II und
R¹⁰ ein o-Phenylen-, Vinylen- oder 1,2-Ethylenrest ist,
verwendet. Auch hier kann die Umsetzung in beliebiger Reihenfolge, aber auch in einer einzigen Stufe durchgeführt werden. Es konnte noch nicht festgestellt werden, ob die Sulfonamidocarbonsäuren der allgemeinen Formel Va oder Vb mit den Alkanolaminen der allgemeinen Formel II zu Sulfonamidocarbonsäureaminoalkylestern, zu Sulfonamidocarbonsäurealkanolamiden oder zu Gemischen derselben umgesetzt werden. Diese Umsetzungsprodukte werden hier der Einfachheit halber immer als Alkanolamide bezeichnet. Die vorgenannten Sulfonamidocarbonsäuren sind z.B. aus der DE-C 28 40 112 und der DE-A 33 04 164 bekannt.

Anschließend kann dann in dem erhaltenen Reaktionsgemisch enthaltenes, überschüssiges Alkanolamin zur Einstellung eines pH-Wertes im Bereich von 4,5 bis 9,5 mit Fettsäuren mit 3 bis 22, vorzugsweise 3 bis 11 Kohlenstoffatomen Ethercarbonsäuren der allgemeinen Formel IV, in der R³, m und q wie oben definiert sind, und/oder Aryl- bzw. Alkylsulfonamidocarbonsäuren der allgemeinen Formel Va bzw. Vb, in denen R⁴, R⁵, R⁶, R⁷, R⁸ und R⁹ wie oben definiert sind, umgesetzt werden.

Es ist bevorzugt, alle Umsetzungen so durchzuführen, daß man das Reaktionsgemisch durchgehend flüssig hält. Man erreicht dies z.B. mit dem bevorzugten, großen Überschuß an Alkanolaminen.

Schließlich können dem Reaktionsgemisch nach der Umsetzung noch die weiter unten beschriebenen Fungizide, vorzugsweise in einer Menge von 1 Gewichtsteil Fungizide auf 10 bis 100 Gewichtsteile der in dem bioziden bzw. biostatischen Gemisch enthaltenen 1,3,5-Triazin-2,4,6-tris-alkylaminocarbonderivate der allgemeinen Formel I, in der R¹ und n wie oben definiert sind, zugesetzt werden.

Ein nach der vorstehend erläuterten Umsetzung vorhandener Überschuß an Alkanolaminen wird, wie oben angegeben, zur Einstellung eines geeigneten pH-Bereichs und unter Bildung weiterer Anteile an Alkanolamiden bzw. Alkanolammoniumsalzen vollständig oder teilweise neutralisiert.

Beispiele für geradkettige oder verzweigte, gesättigte oder ungesättigte Fettsäuren mit 3 bis 22 Kohlenstoffatomen sind Propansäure, die o.g. Fettsäuren mit 5 bis 22 Kohlenstoffatomen sowie technische Gemische derselben. Die Umsetzungsprodukte der Alkanolamine mit den Monocarbonsäuren können weiterhin in den wasserhaltigen Systemen als Korrosionsschutzmittel dienen.

Bevorzugte Beispiele für erfindungsgemäß verwendbare Alkanolamine der allgemeinen Formel II, in der R² wie oben definiert ist, Mono- und Diethanolamin, Mono- und Dipropanolamin, Mono- und Diisopropanolamin, 2-Amino-1-butanol, 2-(2'-Aminoethoxy)-ethanol, 2-Amino-2-methyl-1-propanol und 2-Amino-2-ethyl-1,3-propandiol; wie bereits erwähnt, sind Alkanolamine mit primären Aminogruppen bzw. Gemische derselben mit Alkanolaminen mit sekundären Aminogruppen besonders bevorzugt.

Bevorzugt sind weiterhin auch sekundäre Alkanolamine, die neben einer einzigen Hydroxyalkyl-, Hydroxyalkyl-oxyalkylen- oder Dihydroxyalkylgruppe gemäß den oben für R² angegebenen Definitionen mit einer Alkylgruppe mit 1 bis 6 Kohlenstoffatomen; z.B. Methyl, Ethyl, n-Propyl, i-Propyl, n-Butyl, i-Butyl, Pentyl, Cyclopentyl, Hexyl- oder Cyclohexyl substituiert sind.

Derartige sekundäre Monoalkanol-monoalkylamine sind handelsüblich; typische Vertreter sind Methyl-hydroxyethyl-amin, n-Butyl-hydroxyethylamin und Cyclohexyl-hydroxyethyl-amin sowie die entsprechend substituierten Hydroxypropylderivate. Die von diesen Monoalkanolmonoalkylaminen abgeleiteten Verbindungen der allgemeinen Formel I weisen zum Teil ausgeprägte fungizide Eigenschaften auf, die die Zugabe anderer Fungizide zur Verbesserung der biostatischen Eigenschaften überflüssig machen.

Beispiele für Alkylengruppen mit 4 bis 6 Kohlenstoffatomen, die den Rest R⁶ bilden können, sind Butylen-, Pentylen-, Hexylen-, 2-Methyl-propylen-, 2-Methyl-butylen-, 3-Methyl-butylen-, 2,2-Dimethylpropylen- und 2,2-Dimethyl-butylengruppen.

Die biozide bzw. biostatische Wirkung der erfindungsgemäß verwendeten 1,3,5-Triazin-2,4,6-tris-alkylaminocarbonsäurederivate erstreckt sich auf Bakterien, Hefen und Pilze. Dabei sind die Grenzen zwischen einer bioziden und einer biostatischen Wirkung fließend. Abhängig von der verwendeten Menge und der Einwirkungsdauer überwiegt entweder die biozide (keimabtötende) oder die biostatische (wachstumshemmende) Wirkung. Verwendet man zusätzlich zu den 1,3,5-Triazin-2,4,6-tris-alkylaminocarbonsäurederivaten noch ein Fungizid, treten synergistische Effekte auf, d.h. die Wirkungen verstärken sich gegenseitig. Beispiele für Fungizide sind Pyrithion und dessen Derivate, N-Alkyl- oder N-Aryl-, insbesondere N-Cyclohexyl-, Diazeniumdioxidsalze, z.B. mit Kalium, Aluminium, Zinn oder Kupfer, als Metallkomponente (Ullmanns Encyklopädie der technischen Chemie, 4. Auflage, Band 17, Verlag Chemie, Weinheim 1979, S. 369), Phenole, Kresole, 1,2-Benzisothiazolin-3-on und dessen Derivate sowie 2-Methyl- und 2-Octyl-4-isothiazolin-3-on, wobei halogenfreie Verbindungen bevorzugt sind. Weiterhin werden bevorzugt Fungizide eingesetzt, die wasserlöslich und alkalistabil sind.

Gemäß einer weiteren bevorzugten Ausführungsform der Erfindung werden als Fungizide Pyrithion oder Derivate desselben und/oder N-Alkyl- oder N-Aryl-, insbesondere N-Cyclohexyl-, Diazeniumdioxidsalze, z.B. mit Kalium, Aluminium, Zinn oder Kupfer, als Metallkomponente verwendet. Pyrithion ist die Kurzbezeichnung für 2-Pyridinthiol-1-oxid, das mit 1-Hydroxy-2-pyridinthion im tautomeren Gleichgewicht steht. Als Derivate des Pyrithions kommen die Ammonium-, Natrium-, Magnesium- und Zinksalze sowie 2,2'-Dithiobis(pyridin-1,1'-dioxid), das Disulfid des Pyrithions, in Frage. Das Anion des Pyrithions ist unter Umständen mit Schwermetallen fällbar. Dagegen weisen die o.g. N-Alkyl- bzw. N-Aryl-diazeniumdioxidsalze neben fungiziden Eigenschaften auch komplexierende Eigenschaften auf. Daher wird bevorzugt ein Gemisch von Pyrithion bzw. dessen Derivaten und den o.g. N-Alkyldiazeniumdioxidsalzen verwendet. Es können aber auch nur Pyrithion oder Derivate desselben eingesetzt werden, wobei die fungizide Wirkung in Abwesenheit signifikanter Mengen an Schwermetallen erhalten bleibt. Da Kombinationen der genannten Fungizide mit den erfindungsgemäßen 1,3,5-Triazin-2,4,6-tris-alkylaminocarbonsäurederivaten synergistische Effekte aufweisen, reichen sehr geringe Mengen derselben für die erfindungsgemäße Verwendung in wasserhaltigen Systemen aus.

Gemäß einer weiteren bevorzugten Ausführungsform der Erfindung enthalten die wasserhaltigen Systeme 0,05 bis 0,40 Gew.-% der 1,3,5-Triazin-2,4,6-tris-alkylaminocarbonsäurederivate und 0,0001 bis 0,2 Gew.-%, vorzugsweise 0,001 bis 0,1, insbesondere 0,001 bis 0,02 Gew.-% Fungizide, bezogen auf die Gesamtformulierung.

Es sind Verwendungen in nahezu beliebigen wäßrigen bzw. wasserhaltigen Systemen möglich, z.B. in Metallbearbeitungsflüssigkeiten, Kühlmitteln für Kühlkreisläufe, Reinigern, Hydraulikflüssigkeiten, Kosmetika und Anstrichstoffen. Bei der Verwendung in Kosmetika werden diese bevorzugt nach dem oben beschriebenen Verfahren auf einen pH-Wert im Bereich von 4,5 bis 7,0 eingestellt. Kühlschmierstoffe werden dagegen bevorzugt auf einen pH-Wert im Bereich von 7,5 bis 9,5 eingestellt.

Gemäß einer weiteren bevorzugten Ausführungsform der Erfindung werden die 1,3,5-Triazin-2,4,6-tris-alkylaminocarbonsäurederivate in Kühlschmierstoffen verwendet.

Kühlschmierstoffe sind wäßrige Flüssigkeiten, die z.B. beim Bohren, Mahlen, Fräsen, Drehen, Schneiden, Sägen, Schleifen, Gewindeschneiden oder beim Walzen oder Ziehen von Metallen zum Kühlen und Schmieren verwendet werden. Diese können nach dem Mineralölanteil in drei Gruppen eingeteilt werden:
a) synthetische Kühlschmierstoffe, die mineralölfrei sind,
b) halbsynthetische Kühlschmierstoffe, die ca. 10 bis 60 Gew.-% Mineralöl enthalten und
c) Kühlschmierstoffe, die ca. 60 bis 80 % Mineralöl enthalten.

Die Kühlschmierstoffe können weiterhin Polyglykole enthalten. Anstelle von Mineralölen können auch natürliche oder synthetische Fettsäureester, z.B. Rüböl oder Esteröle, verwendet werden.

Allen drei Typen von Kühlschmierstoffen können weitere Additive wie Korrosionsinhibitoren, Kupfer-Passivatoren, Antiverschleißmittel, Emulgatoren, Trägerstoffe, Fällungsmittel, Sauerstoffabfänger, Komplexierungsmittel oder schaumverhütende Mittel zugesetzt sein.

Beispiele für Korrosionsinhibitoren sind organische Säuren, deren Salze und Ester, z.B. Benzoesäure, p-tert.-Butylbenzoesäure, Dinatriumsebacat, Triethanolamin-laurat, Isononansäure, das Triethanolaminsalz von p-Toluolsulfonamidocapronsäure, Natrium-N-lauroylsarcosinat oder Nonylphenoxyessigsäure oder Polycarbonsäuren; stickstoffhaltige Substanzen, z.B. Fettsäurealkanolamide, Imidazoline, Oxazoline, Triazole, Triethanolamin, Fettamine, N-Acylsarkosine, oder anorganische Nitrite oder Nitrate; phosphorhaltige Substanzen, z.B. Aminphosphate, Phosphonsäuren, Phosphonate, Phosphonocarbonsäuren, Phosphinocarbonsäuren, oder anorganische Phosphate wie NaH₂PO₄, und schwefelhaltige Substanzen, z.B. Salze von Petroleumsulfonaten oder Alkylbenzolsulfonaten, oder heterocyclische Verbindungen, die im Ring ein Schwefelatom oder mehrere enthalten.

Als Kupfer-Passivatoren können z.B. Benztriazole, Methylenbis-benztriazole wie Natrium-2-mercaptobenztriazol, Thiadiazole, z.B. 2,5-Dimercapto-1,3,4-thiadiazol-Derivate, oder Tolyltriazole dienen.

Antiverschleißmittel können AW(Anti-Wear)- oder EP(Extreme-Pressure)-Additive sein, z.B. Schwefel, Phosphor oder Halogen enthaltende Substanzen, wie sulfurierte Fette und Olefine, Tritolylphosphat, Mono- und Diester der Phosphorsäure, Additionsprodukte von Ethylenoxid und/oder Prolylenoxid an Polyhydroxyverbindungen, die gegebenenfalls partiell mit Fettsäuren verestert sind, Chlorparaffine oder ethoxylierte Phosphatester, wobei chlorfreie Verbindungen bevorzugt sind.

Beispiele für Emulgatoren sind Ethercarbonsäuren, Fettsäurealkanolamide, Natrium-Petroleumsulfonate, Mono- oder Diester oder -ether von Polyethylen-, Polypropylen- oder gemischten Polyethylen/Polypropylenglykolen oder Fettsäureseifen.

Als Trägerstoffe können z.B. Poly(meth)acrylsäure und seine Salze, hydrolysiertes Polyacrylnitril, Polyacrylamid und dessen Copolymere, Ligninsulfonsäure und deren Salze, Stärke und Stärkederivate, Cellulose, Alkylphosphonsäuren, 1-Aminoalkyl-1,1-diphosphonsäuren und ihre Salze, Polymaleinsäuren und andere Polycarbonsäuren, Esteröle, natürliche oder synthetische Fettsäureester, z.B. Rüböl, oder Alkaliphosphate verwendet werden.

Beispiele für Fällungsmittel sind Alkaliphosphate oder Alkalicarbonate.

Beispiele für Sauerstoffabfänger sind Alkalisulfate, Morpholin und Hydrazin.

Die erfindungsgemäßen 1,3,5-Triazin-2,4,6-tris-alkylaminocarbonsäurederivate weisen selbst komplexierende Eigenschaften auf: Es können aber auch weitere Komplexierungsmittel, z.B. Phosphonsäurederivate, Nitrilotriessigsäure oder Ethylendiamin-tetraessigsäure und deren Salze, zugesetzt werden. Im übrigen weisen auch die gegebenenfalls als Fungizide einzusetzenden N-Alkyl-bzw. N-Aryldiazeniumdioxidsalze komplexierende Eigenschaften auf, worauf bereits hingewiesen wurde.

Beispiele für schaumverhütende Mittel sind Distearylsebacinsäurediamid, Distearyladipinsäurediamid oder Ethylenoxid- und/oder Propylenoxid-Additionsprodukte solcher Amide, Fettalkohole und deren Ethylenoxid- und/oder Propylenoxid-Additionsprodukte, natürliche und synthetische Wachse, Silikonverbindungen, Kieselsäurederivate und pyrogenes Siliciumdioxid.

Die Erfindung ist demnach weiterhin auf Kühlschmierstoffe gerichtet, die
a) 1,3,5-Triazin-2,4,6-tris-alkylaminocarbonsäurederivate der obigen allgemeinen Formel I, in der R¹ und n wie oben definiert sind,
b) Fungizide,
c) Wasser,
d) gegebenenfalls Mineralöl,
e) gegebenenfalls Emulgatoren und/oder weitere Hilfsstoffe,
f) gegebenenfalls Korrosionsinhibitoren,
enthalten, wobei die 1,3,5-Triazin-2,4,6-tris-alkylaminocarbonsäurederivate in einer Menge von 0,05 bis 0,40 Gew.-% und die Fungizide in einer Menge von 0,0001 bis 0,2 Gew.-%, vorzugsweise 0,001 bis 0,1, insbesondere 0,001 bis 0,02 Gew.-%, bezogen auf die Gesamtmenge des Kühlschmierstoffs, enthalten sind.

Besonders bevorzugt sind Kühlschmierstoffe, die als Emulgatoren und/oder weitere Hilfsstoffe
a) Ethercarbonsäuren der allgemeinen Formel IV, in der R³, m und q wie oben definiert sind, in Form ihrer Alkanolamide und/oder Alkanolammoniumsalze mit Alkanolaminen der allgemeinen Formel II, in der R² wie oben definiert ist,
b) Fettsäurealkanolamide auf der Basis geradkettiger oder verzweigter, gesättigter oder ungesättigter Fettsäuren mit 12 bis 22 Kohlenstoffatomen und Aminen der allgemeinen Formel II,
c) Aryl- bzw. Alkylsulfonamidocarbonsäuren der allgemeinen Formel Va bzw. Vb, in denen R⁴, R⁵, R⁶, R⁷ und R⁸ wie oben definiert sind, in Form ihrer Alkanolamide und/oder Alkanolammoniumsalze mit Alkanolaminen der allgemeinen Formel II, in der R² wie oben definiert ist,
d) geradkettige oder verzweigte, ungesättigte oder gesättigte Carbonsäuren mit 5 bis 22, vorzugsweise 5 bis 11, Kohlenstoffatomen zur Einstellung eines pH-Wertes im Bereich von 7,5 bis 9,5, oder
e) geradkettige oder verzweigte Fettalkohole mit 12 bis 18 Kohlenstoffatomen
enthalten.

Weitere besonders bevorzugte Kühlschmierstoffe enthalten als Fungizide Pyrithion oder Derivate desselben und/oder N-Alkyldiazeniumdioxidsalze.

Die Kühlschmierstoffe der Erfindung können durch Zusammenmischen der Einzelkomponenten hergestellt werden. Falls die Kühlschmierstoffe der Erfindung einen Gehalt an Fettsäurealkanolamiden aufweisen sollen, ist es bevorzugt, die Verbindungen der allgemeinen Formel I in der oben erläuterten Weise in Form ihrer Gemische mit den Fettsäurealkanolamiden herzustellen. Dieses Verfahren bietet zudem den Vorteil, daß ausschließlich flüssige Reaktionsgemische erhalten werden, die ohne weitere Maßnahmen, z.B. eine Zerkleinerung oder Auflösung in geeigneten Lösemitteln, weiterverarbeitet werden können.

Die Erfindung wird im folgenden anhand besonders bevorzugter Ausführungsbeispiele näher erläutert.

Die Beispiele 1 bis 25 zeigen die Herstellung von erfindungsgemäß verwendeten Derivaten des 2,4,6-Tris(omega'-carboxypentylamino)-1,3,5-Triazins, im folgenden kurz Triazincarbonsäure genannt, das kommerziell erhältlich oder durch Umsetzung von Cyanursäurechlorid mit dem Natriumsalz der 6-Aminohexansäure gemäß EP-B 0 046 139 erhalten werden kann.

Die Triazincarbonsäure kann als handelsübliches Produkt oder in Form des handelsüblichen wasserhaltigen Produktes verwendet werden. In den folgenden Beispielen wurde ein ca. 50-Gew.% Wasser enthaltendes, festes Produkt eingesetzt.

### Beispiel 1

75 g (0,714 mol; 26,7 mol pro mol Triazincarbonsäure) Diethanolamin wurden auf 60°C erwärmt und 25 g (0,0267 mol) Triazincarbonsäure unter Rühren zugegeben, bis eine klare Lösung entstand.

Man erhielt 100 g einer klaren, niedrigviskosen Flüssigkeit.

### Beispiel 2

75 g (0,714 mol) Diethanolamin wurden mit 25 g (0,0267 mol) Triazincarbonsäure verrührt. Nach einer Reaktionszeit von mehreren Stunden bei 150 bis 160°C wurden 10 g Wasser abdestilliert.

Die Endsäurezahl betrug 10 mg KOH/g.

Man erhielt 90 g einer klaren, mittelviskosen Flüssigkeit.

### Beispiel 3

75 g (1,230 mol; 46,1 mol pro mol Triazincarbonsäure) Monoethanolamin wurden auf 60°C erwärmt und 25 g (0,0267 mol) Triazincarbonsäure unter Rühren zugegeben, bis eine klare Lösung entstand.

Man erhielt 100 g einer klaren, niedrigviskosen Flüssigkeit.

### Beispiel 4

75 g (1,230 mol) Monoethanolamin wurden bei 60°C mit 25 g (0,0267 mol) Triazincarbonsäure verrührt und auf 140 bis 143°C erhitzt. Nach einer Reaktionszeit von 10 Stunden wurden 18 g Wasser abdestilliert.

Die Endsäurezahl betrug 12 mg KOH/g.

Man erhielt ein weißes, festes Produkt.

### Beispiel 5

75 g (0,843 mol; 31,5 mol pro mol Triazincarbonsäure) 2-Amino-1-butanol wurden auf 60°C erwärmt und 25 g (0,0267 mol) Triazincarbonsäure unter Rühren zugegeben, bis eine klare Lösung entstand.

Man erhielt 100 g einer klaren, niedrigviskosen Flüssigkeit.

### Beispiel 6

863 g (9,697 mol; 31,5 mol pro mol Triazincarbonsäure) 2-Amino-1-butanol wurden bei 60°C mit 287 g (0,307 mol) Triazincarbonsäure verrührt und auf 145°C erhitzt. Nach einer Reaktionszeit von 20 Stunden wurden 150 g Wasser abdestilliert.

Die Endsäurezahl betrug 10 mg KOH/g.

Man erhielt 1000 g einer klaren, niedrigviskosen Flüssigkeit.

### Beispiel 7

75 g (0,714 mol; 26,7 mol pro mol Triazincarbonsäure) 2-(2'-Aminoethoxy)-ethanol wurden bei 60°C mit 25 g (0,0267 mol) Triazincarbonsäure verrührt, bis eine klare Lösung entstand.

Man erhielt 100 g einer klaren, niedrigviskosen Flüssigkeit.

### Beispiel 8

375 g (3,571 mol; 26,7 mol pro mol Triazincarbonsäure) 2-(2'-Aminoethoxy)-ethanol wurden bei 60°C mit 125 g (0,134 mol) Triazincarbonsäure verrührt und auf 145°C erhitzt. Nach einer Reaktionszeit von 16 Stunden wurden 73 g Wasser abdestilliert.

Die Endsäurezahl betrug 7 mg KOH/g.

Man erhielt 427 g eines weißen, pastösen Produkts.

### Beispiel 9

20 g (0,190 mol; 7,1 mol pro mol Triazincarbonsäure) 2-(2'-Aminoethoxy)-ethanol und 55 g (0,618 mol; 23,1 mol pro mol Triazincarbonsäure) 2-Amino-1-butanol wurden auf 60°C erwärmt und mit 25 g (0,0267 mol) Triazincarbonsäure verrührt, bis eine klare Lösung entstand.

Man erhielt 100 g einer klaren, niedrigviskosen Flüssigkeit.

### Beispiel 10

228 g (2,171 mol; 7,1 mol pro mol Triazincarbonsäure) 2-(2'-Aminoethoxy)-ethanol und 627 g (7,045 mol; 23,1 mol pro mol Triazincarbonsäure) 2-Amino-1-butanol wurden bei 60°C mit 285 g (0,304 mol) Triazincarbonsäure verrührt und auf 145°C erhitzt. Nach einer Reaktionszeit von 16 Stunden wurden 140 g Wasser abdestilliert.

Die Endsäurezahl betrug 13 mg KOH/g.

Man erhielt 1000 g einer klaren, mittelviskosen Flüssigkeit.

### Beispiel 11

75 g (0,758 mol; 28,4 mol pro mol Triazincarbonsäure) AMP 90 (2-Amino-2-methyl-1-propanol mit 10 % Wasser) wurden auf 60°C erwärmt und mit 25 g (0,0267 mol) Triazincarbonsäure verrührt, bis eine klare Lösung entstand.

Man erhielt eine klare, niedrigviskose Flüssigkeit.

### Beispiel 12

833 g (8,424 mol; 31,5 mol pro mol Triazincarbonsäure) AMP 90 wurden auf 60°C erwärmt, mit 250 g (0,267 mol) Triazincarbonsäure verrührt und auf 140 bis 145°C erhitzt. Nach einer Reaktionszeit von 20 Stunden wurden 240 g Wasser abdestilliert.

Die Endsäurezahl betrug 15 mg KOH/g.

Man erhielt 843 g eines fast klaren, hochviskosen Produkts.

### Beispiel 13

75 g (0,630 mol; 23,6 mol pro mol Triazincarbonsäure) AEPD (2-Amino-2-ethyl-1,3-propandiol) wurden auf 60°C erwärmt und mit 25 g (0,0267 mol) Triazincarbonsäure verrührt, bis eine klare Lösung entstand.

Man erhielt 100 g einer klaren, mittelviskosen Flüssigkeit.

### Beispiel 14

990 g (8,319 mol; 23,6 mol pro mol Triazincarbonsäure) AEPD wurden auf 60°C erwärmt, mit 330 g (0,353 mol) Triazincarbonsäure verrührt und auf 140 bis 145°C erhitzt. Nach einer Reaktionszeit von 16 Stunden wurden 320 g Wasser abdestilliert.

Die Endsäurezahl betrug 10 mg KOH/g.

Man erhielt 1000 g einer klaren, hochviskosen Flüssigkeit.

### Beispiel 15

75 g (1,000 mol; 37,4 mol pro mol Triazincarbonsäure) Monoisopropanolamin wurden auf 60°C erwärmt und mit 25 g (0,0267 mol) Triazincarbonsäure verrührt, bis eine klare Lösung entstand.

Man erhielt 100 g einer niedrigviskosen Flüssigkeit.

### Beispiel 16

375 g (5,000 mol; 37,4 mol pro mol Triazincarbonsäure) Monoisopropanolamin wurden auf 60°C erwärmt, mit 125 g (0,134 mol) Triazincarbonsäure verrührt und auf 140°C erhitzt. Nach einer Reaktionszeit von 16 Stunden wurden 74 g Wasser abdestilliert.

Die Endsäurezahl betrug 12 mg KOH/g.

Man erhielt 426 g einer klaren, niedrigviskosen Flüssigkeit.

### Beispiel 17

75 g (0,503 mol; 18,9 mol pro mol Triazincarbonsäure) Triethanolamin wurden auf 60°C erwärmt und mit 25 g (0,0267 mol) Triazincarbonsäure verrührt, bis eine klare Lösung entstand.

Man erhielt 100 g einer klaren, niedrigviskosen Flüssigkeit.

### Beispiel 18

750 g (5,034 mol; 18,9 mol pro mol Triazincarbonsäure) Triethanolamin wurden auf 60°C erwärmt, mit 250 g (0,267 mol) Triazincarbonsäure verrührt und auf 140 bis 145°C erhitzt. Nach einer Reaktionszeit von 16 Stunden wurden 130 g Wasser abdestilliert.

Die Endsäurezahl betrug 6 mg KOH/g.

Man erhielt 870 g einer klaren, mittelviskosen Flüssigkeit.

### Beispiel 19

8 Teile Triazincarbonsäure und 1 Teil 2-(8-Heptadecenyl)-4,5-dihydro-1-(2-hydroxyethyl)-imidazol wurden bei Umgebungstemperatur unter Rühren zu 6 Gewichtsteilen Monoisopropanolamin gegeben. Es wurde gerührt, bis sich eine klare, bernsteinfarbene Flüssigkeit gebildet hatte.

### Beispiel 20

### 1. Stufe

130 g (1,238 mol; 4,8 mol pro mol Triazincarbonsäure) 2-(2'-Aminoethoxy)-ethanol und 370 g (4,157 mol; 16,2 mol pro mol Triazincarbonsäure) 2-Amino-1-butanol wurden mit 190 g (0,674 mol; 2,6 mol pro mol Triazincarbonsäure) Olein bei 145°C umgesetzt. Nach einer Reaktionszeit von 10 Stunden wurden 12 g Wasser abdestilliert.

Man erhielt 678 g eines flüssigen Produkts mit einer Säurezahl von 7 mg KOH/g.

### 2. Stufe

678 g des Produkts der Stufe 1 wurden auf 70°C erwärmt und mit 240 g (0,256 mol) Triazincarbonsäure verrührt.

Man erhielt eine klare, viskose Flüssigkeit.

### Beispiel 20a

### 1. Stufe

130 g (1,238 mol) 2-(2'-Aminoethoxy)-ethanol und 370 g (4,157 mol) 2-Amino-1-butanol wurden mit 190 g (0,674 mol) Olein bei 145°C umgesetzt. Nach einer Reaktionszeit von 10 Stunden wurden 12 g Wasser abdestilliert.

Man erhielt 678 g eines flüssigen Produkts mit einer Säurezahl von 7 mg KOH/g.

### 2. Stufe

678 g des Produkts der Stufe 1 wurden auf 70°C erwärmt und mit 240 g (0,256 mol) Triazincarbonsäure und 174 g (0,497 mol; 1,9 mol pro mol Triazincarbonsäure) einer handelsüblichen Arylsulfonamidocarbonsäure mit einem Molekulargewicht von ca. 350 (Hostacor^{R} H flüssig), im folgenden kurz Sulfonamidocarbonsäure genannt, verrührt.

Man erhielt eine stabile, leicht trübe Flüssigkeit.

### Beispiel 20b

### 1. Stufe

130 g (1,238 mol) 2-(2'-Aminoethoxy)-ethanol und 370 g (4,157 mol) 2-Amino-1-butanol wurden mit 190 g (0,674 mol) Olein bei 145°C umgesetzt. Nach einer Reaktionszeit von 10 Stunden wurden 12 g Wasser abdestilliert.

Man erhielt 678 g eines flüssigen Produkts mit einer Säurezahl von 7 mg KOH/g.

### 2. Stufe

678 g des Produkts der Stufe 1 wurden auf 70°C erwärmt und mit 240 g (0,0256 mol) Triazincarbonsäure und 106 g (0,148 mol; 0,6 mol pro mol Triazincarbonsäure) Ethercarbonsäure (handelsübliches Umsetzungsprodukt von 1 mol Chloressigsäure mit einem Kondensationsprodukt von 1 mol eines technischen Oleylalkohols mit 10 mol Ethylenoxid) verrührt.

Man erhielt eine klare, viskose Flüssigkeit.

### Beispiel 20c

130 g (1,238 mol) 2-(2'-Aminoethoxy)-ethanol und 370 g (4,157 mol) 2-Amino-1-butanol wurden auf 70°C erwärmt und mit 174 g (0,497 mol) Sulfonamidocarbonsäure und 240 g (0,256 mol) Triazincarbonsäure verrührt.

Man erhielt eine helle, klare, mittelviskose Flüssigkeit.

### Beispiel 20d

130 g (1,238 mol) 2-(2'-Aminoethoxy)-ethanol, 370 g (4,157 mol) 2-Amino-1-butanol, 174 g (0,497 mol) Sulfonamidocarbonsäure und 240 g (0,256 mol) Triazincarbonsäure wurden bei 145°C umgesetzt. Nach einer Reaktionszeit von 12 Stunden wurden 143 g Wasser abdestilliert.

Man erhielt 771 g eines mittelviskosen, klaren, flüssigen Produkts mit einer Endsäurezahl von 25 mg KOH/g.

### Beispiel 20e

130 g (1,238 mol) 2-(2'-Aminoethoxy)-ethanol und 370 g (4,157 mol) 2-Amino-1-butanol wurden auf 70°C erwärmt und mit 106 g (0,148 mol) Ethercarbonsäure und 240 g (0,256 mol) Triazincarbonsäure verrührt.

Man erhielt eine helle, mittelviskosen Flüssigkeit.

### Beispiel 20f

130 g (1,238 mol) 2-(2'-Aminoethoxy)-ethanol, 370 g (4,157 mol) 2-Amino-1-butanol, 106 g (0,148 mol) Ethercarbonsäure und 240 g (0,256 mol) Triazincarbonsäure wurden bei 145°C umgesetzt. Nach einer Reaktionszeit von 13 Stunden wurden 137 g Wasser abdestilliert.

Man erhielt 709 g eines klaren, mittelviskosen Produkts mit einer Endsäurezahl von 20 mg KOH/g.

### Beispiel 21

678 g der Flüssigkeit der ersten Stufe von Beispiel 20 wurden auf 60°C erwärmt, mit 240 g (0,256 mol) Triazincarbonsäure verrührt und auf 140 bis 150°C erhitzt. Nach einer Reaktionszeit von 10 Stunden wurden 138 g Wasser abdestilliert.

Die Endsäurezahl betrug 14 mg KOH/g.

Man erhielt 780 g eines klaren, mittelviskosen Produkts.

### Beispiel 22 (nicht erfindungsgemäß)

273 g (1,019 mol; 2,8 mol pro mol Triazincarbonsäure) eines handelsüblichen technischen Oleylalkohols (ca. 90%-ig, Jodzahl ca. 95) wurden auf 80 bis 100°C erwärmt, langsam mit 333 g (0,356 mol) Triazincarbonsäure verrührt und bis maximal 200°C erhitzt. Nach einer Reaktionszeit von ca. 20 Stunden wurden 206 ml Wasser abdestilliert.

Die Endsäurezahl betrug 18 mg KOH/g.

Man erhielt eine klare, mittelviskose Flüssigkeit.

### Beispiel 23 (nicht erfindungsgemäß)

350 g (2,692 mol; 15 mol pro mol Triazincarbonsäure) 2-Ethylhexanol wurden auf 100°C erwärmt, mit 168 g (0,179 mol) Triazincarbonsäure verrührt und langsam bis maximal 190°C erhitzt. Nach einer Reaktionszeit von 10 Stunden wurden 358 g Wasser und Ethylhexanol abdestilliert.

Die Endsäurezahl betrug 15 mg KOH/g.

Man erhielt 160 g einer klaren, viskosen Flüssigkeit.

### Beispiel 24 (nicht erfindungsgemäß)

273 g (3,592 mol; 10,1 mol pro mol Triazincarbonsäure) 1,2-Propylenglykol wurden auf 100°C erwärmt, mit 333 g (0,356 mol) Triazincarbonsäure verrührt und auf maximal 168°C erhitzt. Nach einer Reaktionszeit von 7 Stunden wurden 276 ml Wasser abdestilliert.

Die Endsäurezahl betrug 38 mg KOH/g.

Man erhielt 330 g einer viskosen, hellen Flüssigkeit.

### Beispiel 25 (nicht erfindungsgemäß)

324 g (3,000 mol; 8,4 mol pro mol Triazincarbonsäure) Benzylalkohol wurden mit 333 mg (0,356 mol) Triazincarbonsäure verrührt und unter Stickstoff auf maximal 180°C erhitzt. Nach einer Reaktionszeit von 9 Stunden wurden 180 ml Wasser abdestilliert.

Die Endsäurezahl betrug 21 mg KOH/g.

Man erhielt 477 g einer niedrigviskosen Flüssigkeit.

### Beispiel 26

375 g (3,2 mol; 23,97 mol pro mol Triazincarbonsäure) N-Butylethanolamin wurden mit 125 g (0,13 mol) Triazincarbonsäure bei 145°C umgesetzt. Nach einer Reaktionszeit von 14 Stunden wurden 85 g Wasser abdestilliert.

Man erhielt 415 g eines flüssigen Produkts mit einer Säurezahl von 13 mg KOH/g.

### Beispiel 27

167 g (1,43 mol; 8 mol pro mol Triazincarbonsäure) N-Butylethanolamin wurden 167 g (0,18 mol) Triazincarbonsäure bei 148°C umgesetzt. Nach einer Reaktionszeit von 14 Stunden wurden 56 g Wasser abdestilliert.

Man erhielt 278 g eines zähflüssigen Produkts mit einer Säurezahl von 7 mg KOH/g.

### Beispiel 28

100 g (0,85 mol; 2 mol pro mol Triazincarbonsäure) N-Butylethanolamin und 100 g (0,95 mol; 2,22 mol pro mol Triazincarbonsäure) 2-(2'-Aminoethoxy)-ethanol und 100 g (1,33 mol; 3,11 mol pro mol Triazincarbonsäure) Monoisopropanolamin wurden mit 200 g (0,21 mol) Triazincarbonsäure bei 150°C umgesetzt. Nach einer Reaktionszeit von 10 Stunden wurden 65 g Wasser abdestilliert.

Man erhielt 435 g eines mittelviskosen Produkts mit einer Säurezahl von 14 mg KOH/g.

### Beispiel 29

516 g (4,9 mol; 8,75 mol pro mol Triazincarbonsäure) 2-(2'-Aminoethoxy)-ethanol und 248 g (3,3 mol; 5,89 mol pro mol Triazincarbonsäure) Monoisopropanolamin wurden mit 526 g (0,56 mol) Triazincarbonsäure bei 150°C umgesetzt. Nach einer Reaktionszeit von 14 Stunden wurden 290 g Wasser abdestilliert.

Man erhielt 1000 g eines flüssigen Produkts mit einer Säurezahl von 11 mg KOH/g.

### Beispiel 30

### 1. Stufe

117 g (1 mol) N-Butylethanolamin wurden auf 110°C erwärmt und langsam unter Kühlung 148 g (1 mol) Phthalsäureanhydrid zugegeben. Die Reaktionszeit dauerte 4 Stunden bei maximal 130°C.

Man erhielt 265 g eines festen, glasartigen Produkts mit einer Säurezahl von 211 mg KOH/g.

### 2.Stufe

Bei 100°C wurden 50 g der Stufe 1 mit 50 g Produkt aus Beispiel 10 vermischt.

Man erhält ein viskoses Produkt mit einer Säurezahl von 115 mg KOH/g.

### Beispiel 31

50 g Korantin PA (Phthalsäure-2-methyl-2-ethylhexylamid) wurden bei 50°C mit 50 g Produkt aus Beispiel 10 gemischt.

Man erhielt ein viskoses Produkt mit einer Säurezahl von 88 mg KOH/g.

### Vergleichsbeispiele 1 bis 3 und Beispiele 32 bis 54

Eine Reihe der so hergestellten 1,3,5-Triazin-2,4,6-tris-alkylaminocarbonsäurederivate wurde mit Wasser, Spindelöl und weiteren, jeweils angegebenen Zusätzen sowie in einigen Beispielen mit Fungiziden zu Gemischen formuliert, die in einer Verdünnung mit Wasser von 1:20 bis 1:80 Kühlschmierstoffe ergeben.

Weiterhin wurden in den Vergleichsbeispielen 1 und 2 Gemische ohne Biozide und in dem Vergleichsbeispiel 3 ein Gemisch mit einem Borsäurealkanolamin-Kondensationsprodukt als biozides Mittel formuliert.

Angaben in % beziehen sich im folgenden immer auf Gewichtsteile.

Die Angaben in der Spalte "Erl." beziehen sich auf die Erläuterungen der Tabelle 1. Sämtliche in der Tabelle 1 aufgeführten Chemikalien sind im Handel erhältlich.

**Tabelle 1**

| **ERLÄUTERUNGEN** | |
|---|---|
| 1 | Tallöldestillat mit 25-30% Harz (Säurezahl 155-190) |
| 2 | a) Isononansäure |
| | b) 2,2-Dimethyl-octansäure |
| 3 | Spindelöl, Viskosität: 22 mm²/s bei 40°C |
| 4 | a) Umsetzungsprodukt von 1 mol Chloressigsäure mit einem Kondensationsprodukt von 1 mol eines technischen Oleylalkohols mit 10 mol Ethylenoxid (Ethercarbonsäure) |
| | b) Umsetzungsprodukt von 1 mol Chloressigsäure mit einem Kondensationsprodukt von 1 mol C₉- bis C₁₃-Oxoalkohole mit 3 mol Ethylenoxid und 2 mol Propylenoxid (Ethercarbonsäure) |
| 5 | a) technischer Oleylalkohol (ca. 90%-ig, Jodzahl ca. 95) |
| | b) 2-Hexyl-decanol |
| 6 | a) Kondensationsprodukt von 1 mol eines technischen Gemisches von Oleyl- und Cetylalkohol mit 5 mol Ethylenoxid |
| | b) Fettalkohol-polyglykolether (Emulsogen^{R} LP) |
| 7 | a) Kondensationsprodukt von 40 Gewichtsteilen Diethanolamin mit 60 Gewichtsteilen Olein |
| | b) wie a) unter Zusatz von 20% Ethanolamin, bezogen auf die Gesamtmenge von Kondensationsprodukt und Ethanolamin |
| 8 | a) Diethylenglykol |
| | b) Butyldiglykol |
| | c) Butylglykol |
| 9 | a) Natriumpetroleumsulfonat mit einem Molekulargewicht von ca. 460 |
| | b) Natriumalkylbenzolsulfonat mit einem Molekulargewicht von ca. 350 |
| 10 | 50 %-ige Kalilauge |
| 11 | Fungizides Gemisch aus |
| | 10 % Natriumsalz des Pyrithions |
| | 10 % N-(Cylohexyl-diazeniumdioxid)-Kalium-Hydrat in Form |
| | einer 30 %-igen wäßrigen Lösung |
| | 10 % Propylenglykol |
| | 70 % demineralisiertem Wasser |
| 12 | Kondensationsprodukt von 1 mol Borsäure mit 3 mol Ethanolamin |
| 13 | Arylsulfonamidocarbonsäure mit einem Molekulargewicht von ca. 350 (Hostacor^{R} H flüssig; Säuregehalt ca. 90 %-ig, Rest Lösevermittler) |

| Vergleichsbeispiel 1 | Erl. |
|---|---|
| 7 % Fettsäuren | 1 |
| 2 % Sulfonate | 9b) |
| 5 % Fettsäurealkanolamide | 7b) |
| 2 % Hilfsstoffe | 8a) |
| 1 % Hilfsstoffe | 10 |
| 83 % Spindelöl | 3 |

| Vergleichsbeispiel 2 | Erl. |
|---|---|
| 8 % Fettsäuren | 1 |
| 17 % Sulfonate | 9a) |
| 4 % Fettsäurealkanolamide | 7a) |
| 3 % Hilfsstoffe | 8c) |
| 2 % Hilfsstoffe | 10 |
| 36 % Spindelöl | 3 |
| 30 % Wasser | |

| Vergleichsbeispiel 3 | Erl. |
|---|---|
| 20 % Borsäureprodukt | 12 |
| 10 % Fettsäuren | 1 |
| 10 % Fettsäurealkanolamide | 7a) |
| 10 % Hilfsstoffe | 8b) |
| 20 % Spindelöl | 3 |
| 30 % Wasser | |

| Beispiel 32 | Erl. |
|---|---|
| 25 % Beispiel 1 | |
| 31 % Spindelöl | 3 |
| 10 % Fettsäuren | 1 |
| 5 % Fettsäuren | 2b) |
| 4 % Ethercarbonsäuren | 4b) |
| 6 % Fettalkohole | 5a) |
| 5 % nichtionische Emulgatoren | 6a) |
| 1 % Fungizide | 11 |
| 13 % Wasser | |

| Beispiel 33 | Erl. |
|---|---|
| 25 % Beispiel 2 | |
| 31 % Spindelöl | 3 |
| 10 % Fettsäuren | 1 |
| 5 % Fettsäuren | 2b) |
| 4 % Ethercarbonsäuren | 4b) |
| 6 % Fettalkohole | 5a) |
| 14 % Wasser | |
| 5 % nichtionische Emulgatoren | 6a) |

| Beispiel 34 | Erl. |
|---|---|
| 25 % Beispiel 4 | |
| 31 % Spindelöl | 3 |
| 10 % Fettsäuren | 1 |
| 5 % Fettsäuren | 2b) |
| 4 % Ethercarbonsäuren | 4b) |
| 6 % Fettalkohole | 5a) |
| 5 % nichtionische Emulgatoren | 6a) |
| 14 % Wasser | |

| Beispiel 35 | Erl. |
|---|---|
| 22 % Beispiel 6 | |
| 11 % Fettsäuren | 2a) |
| 17 % Fettsäuren | 1 |
| 6 % Ethercarbonsäuren | 4a) |
| 9 % Fettsäurealkanolamide | 7a) |
| 4 % Fettalkohole | 5a) |
| 22 % Spindelöl | 3 |
| 8 % Wasser | |
| 1 % Fungizide | 11 |

| Beispiel 36 | Erl. |
|---|---|
| 21 % Beispiel 10 | |
| 21 % Fettsäuren | 1 |
| 11 % Fettsäuren | 2a) |
| 7 % Ethercarbonsäuren | 4a) |
| 5 % Fettalkohole | 5b) |
| 20 % Spindelöl | 3 |
| 13 % Wasser | |
| 2 % Fungizide | 11 |

| Beispiel 37 | Erl. |
|---|---|
| 25 % Beispiel 14 | |
| 31 % Spindelöl | 3 |
| 10 % Fettsäuren | 1 |
| 3 % Fettsäuren | 2a) |
| 8 % Fettalkohole | 5a) |
| 5 % nichtionische Emulgatoren | 6a) |
| 4 % Ethercarbonsäuren | 4b) |
| 1 % Fungizide | 11 |
| 13 % Wasser | |

| Beispiel 38 | Erl. |
|---|---|
| 25 % Beispiel 16 | |
| 31 % Spindelöl | 3 |
| 10 % Fettsäuren | 1 |
| 5 % Fettsäuren | 2b) |
| 4 % Ethercarbonsäuren | 4b) |
| 6 % Fettalkohole | 5a) |
| 5 % nichtionische Emulgatoren | 6a) |
| 1 % Fungizide | 11 |
| 13 % Wasser | |

| Beispiel 39 | Erl. |
|---|---|
| 35 % Beispiel 17 | |
| 20 % Spindelöl | 3 |
| 10 % Fettsäuren | 1 |
| 5 % Fettsäuren | 2a) |
| 3 % Ethercarbonsäuren | 4b) |
| 6 % Fettalkohole | 5b) |
| 5 % nichtionische Emulgatoren | 6a) |
| 16 % Wasser | |

| Beispiel 40 | Erl. |
|---|---|
| 19 % Beispiel 21 | |
| 29 % Fettsäuren | 1 |
| 29 % Spindelöl | 3 |
| 5 % Hilfsstoffe | 8b) |
| 3 % nichtionische Emulgatoren | 6b) |
| 1 % Fungizide | 11 |
| 14 % Wasser | |

| Beispiel 41 | Erl. |
|---|---|
| 20 % Beispiel 3 | |
| 20 % Fettsäuren | 1 |
| 16 % Fettsäuren | 2a) |
| 7 % Ethercarbonsäuren | 4a) |
| 5 % Fettalkohole | 5b) |
| 18 % Spindelöl | 3 |
| 2 % Fungizide | 11 |
| 12 % Wasser | |

| Beispiel 42 | Erl. |
|---|---|
| 21 % Beispiel 9 | |
| 21 % Fettsäuren | 1 |
| 11 % Fettsäuren | 2a) |
| 7 % Ethercarbonsäuren | 4a) |
| 5 % Fettalkohole | 5b) |
| 20 % Spindelöl | 3 |
| 2 % Fungizide | 11 |
| 13 % Wasser | |

| Beispiel 43 (nicht erfindungsgemäß) | Erl. |
|---|---|
| 7 % Beispiel 25 | |
| 93 % Vergleichsbeispiel 1 | |

| Beispiel 44 (nicht erfindungsgemäß) | Erl. |
|---|---|
| 7 % Beispiel 25 | |
| 91 % Vergleichsbeispiel 1 | |
| 2 % Fungizide | 11 |

| Beispiel 45 (nicht erfindungsgemäß) | Erl. |
|---|---|
| 4 % Beispiel 23 | |
| 96 % Vergleichsbeispiel 1 | |

| Beispiel 46 (nicht erfindungsgemäß) | Erl. |
|---|---|
| 4 % Beispiel 23 | |
| 94 % Vergleichsbeispiel 1 | |
| 2 % Fungizide | 11 |

| Beispiel 47 (nicht erfindungsgemäß) | Erl. |
|---|---|
| 4 % Beispiel 23 | |
| 96 % Vergleichsbeispiel 2 | |

| Beispiel 48 (nicht erfindungsgemäß) | Erl. |
|---|---|
| 4 % Beispiel 23 | |
| 94 % Vergleichsbeispiel 2 | |
| 2 % Fungizide | 11 |

| Beispiel 49 | Erl. |
|---|---|
| 24 % Beispiel 20a | |
| 22 % Fettsäuren | 1 |
| 29 % Spindelöl | 3 |
| 4 % Hilfsstoffe | 8b) |
| 2 % Monoethanolamin | |
| 4 % nichtionische Emulgatoren | 6a) |
| 1 % Fungizide | 11 |
| 14 % Wasser | |

| Beispiel 50 | Erl. |
|---|---|
| 25 % Beispiel 20b | |
| 23 % Fettsäuren | 1 |
| 29 % Spindelöl | 3 |
| 3 % Hilfsstoffe | 8b) |
| 4 % nichtionische Emulgatoren | 6a) |
| 1 % Fungizide | 11 |
| 14 % Wasser | |
| 1 % Monoethanolamin | |

| Beispiel 51 | Erl. |
|---|---|
| 23 % Beispiel 20c | |
| 28 % Fettsäuren | 1 |
| 1 % Fettalkohole | 5a) |
| 4 % nichtionische Emulgatoren | 6a) |
| 4 % Hilfsstoffe | 8b) |
| 1 % Fungizide | 11 |
| 27 % Spindelöl | 3 |
| 12 % Wasser | |

| Beispiel 52 | Erl. |
|---|---|
| 23 % Beispiel 20d | |
| 28 % Fettsäuren | 1 |
| 25 % Spindelöl | 3 |
| 5 % nichtionische Emulgatoren | 6b) |
| 2 % Fettalkohole | 5b) |
| 2 % Hilfsstoffe | 8b) |
| 1 % Fungizide | 11 |
| 14 % Wasser | |

| Beispiel 53 | Erl. |
|---|---|
| 23 % Beispiel 20e | |
| 28 % Fettsäuren | 1 |
| 1 % Fettalkohole | 5a) |
| 4 % nichtionische Emulgatoren | 6a) |
| 4 % Hilfsstoffe | 8b) |
| 1 % Fungizid | 11 |
| 27 % Spindelöl | 3 |
| 12 % Wasser | |

| Beispiel 54 | Erl. |
|---|---|
| 23 % Beispiel 20f | |
| 28 % Fettsäuren | 1 |
| 1 % Fettalkohol | 5a) |
| 4 % nichtionische Emulgatoren | 6a) |
| 4 % Hilfsstoffe | 8b) |
| 1 % Fungizide | 11 |
| 27 % Spindelöl | 3 |
| 12 % Wasser | |

| Beispiel 55 | Erl. |
|---|---|
| 21 % Beispiel 32 | |
| 21 % Fettsäuren | 1 |
| 20 % Spindelöl | 3 |
| 15,5 % Wasser | |
| 1,5 % Ethercarbonsäuren | 4b) |
| 2 % Ethercarbonsäuren | 4a) |
| 2 % nichtionische Emulgatoren | 6b) |
| 5 % Fettalkohole | 5b) |
| 11 % Fettsäuren | 2a) |
| 1 % Monoethanolamin | |

| Beispiel 55a | Erl. |
|---|---|
| 98 % Beispiel 55 | |
| 2 % Fungizide | 11 |

| Beispiel 56 | Erl. |
|---|---|
| 10 % Beispiel 33 | |
| 30 % Spindelöl | 3 |
| 22 % Wasser | |
| 15 % Fettsäuren | 1 |
| 5 % Fettsäuren | 2a) |
| 5 % Fettalkohole | 5b) |
| 2 % Ethercarbonsäuren | 4b) |
| 4 % Ethercarbonsäuren | 4a) |
| 3 % Monoethanolamin | |
| 4 % Fettsäurealkanolamid | 7a) |

| Beispiel 56a | Erl. |
|---|---|
| 98 % Beispiel 56 | |
| 2 % Fungizide | 11 |

| Beispiel 57 | Erl. |
|---|---|
| 10 % Beispiel 34 | |
| 23 % Fettsäuren | |
| 9 % Monoethanolamin | 2a) |
| 2 % Ethercarbonsäuren | 4b) |
| 6 % Fettsäurealkanolamid | 7a) |
| 50 % Wasser | |

| Beispiel 57a | Erl. |
|---|---|
| 98 % Beispiel 57 | |
| 2 % Fungizide | 11 |

| Beispiel 58 | Erl. |
|---|---|
| 5 % Beispiel 33 | |
| 5 % Beispiel 35 | |
| 23 % Fettsäuren | 2a) |
| 10 % Monoethanolamin | |
| 2,5 % Ethercarbonsäuren | 4b) |
| 6 % Fettsäurealkanolamid | 7a) |
| 48,5 % Wasser | |

| Beispiel 58a | Erl. |
|---|---|
| 98 % Beispiel 58 | |
| 2 % Fungizide | 11 |

| Beispiel 59 | Erl. |
|---|---|
| 20 % Beispiel 37 | |
| 25 % Spindelöl | 3 |
| 17 % Fettsäuren | 1 |
| 4 % Fettalkohole | 5b) |
| 4 % nichtionische Emulgatoren | 6b) |
| 1 % Monoethanolamin | |
| 29 % Wasser | |

| Beispiel 59a | Erl. |
|---|---|
| 98 % Beispiel 60 | |
| 2 % Fungizide | 11 |

| Beispiel 60 | Erl. |
|---|---|
| 20 % Beispiel 37 | |
| 25 % Spindelöl | 3 |
| 17 % Fettsäuren | 1 |
| 4 % Fettalkohole | 5b) |
| 4 % nichtionische Emulgatoren | 6b) |
| 1 % Monoethanolamin | |
| 29 % Wasser | |

| Beispiel 60a | Erl. |
|---|---|
| 98 % Beispiel 60 | |
| 2 % Fungizide | 11 |

### Mikrobiologisches Testverfahren

Es wurde ein selbstentwickelter Impfzyklentest durchgeführt. Dazu wurden folgende Verdünnungen der Formulierungen der Vergleichsbeispiele 1 bis 3 und der Beispiele 26 bis 42 mit Hamburger Stadtwasser angesetzt: 1,25 %, 2,5 % und 5,0 % (entspricht 1:80, 1:40 und 1:20).

Die Proben wurden mit einer konzentrierten Mischkeimflora mehrfach beimpft. Die Keimflora enthielt Bakterien, Hefen und Pilze aus laufenden Emulsionssystemen unterschiedlicher Herkunft. Ihre Gesamtkeimzahl betrug ca. 10⁷ Keime/ml.

Die Menge an Mischkeimflora bei der Beimpfung der Proben entsprach der sechsfachen Menge, die nach DAB 9 (Deutsches Arzneibuch) vorgeschlagen wird. Es wurden je 100 ml Probe 6 ml Keimflora verwendet.

Die Proben wurden (in Anlehnung an K.H. Wallhäußer; Praxis der Sterilisation-Desinfektion-Konservierung-Keimidentifizierung, 4. Auflage, Georg Thieme Verlag, Stuttgart 1988) nach diesem Verfahren wiederholt beimpft (maximal 6 Beimpfungen), bis eine antimikrobielle Wirkung nicht mehr feststellbar war. Erfahrungsgemäß entsprach 1 Beimpfung 3 Impfzyklen nach der DAB-9/Wallhäuser-Methode.

Dieses Verfahren hat folgende Vorteile:
1. Es wird eine Mischkeimflora eingesetzt, wie sie in der Praxis vorkommt.
2. Die Proben werden mehrfach einer massiven Keimbelastung ausgesetzt.
3. Die Methode ist schnell und damit industriegerecht. Im Vergleich zur herkömmlichen Methode, die oft mehrere Monate dauert, liegen die Ergebnisse in max. 8 Wochen vor, wenn sie nicht wiederholt werden müssen.
4. Aus den Ergebnissen lassen sich Rückschlüsse ziehen auf die Standzeiten der Gebrauchsemulsionen in den Zentralsystemen.

Die Einwirkzeit der Mikroorganismen auf die Proben betrug ca. 1 Woche. Nach dieser Zeit wurden die Proben auf je zwei Spezialnährböden ausgestrichen und bebrütet. Anschließend wurde unter dem Mikroskop die Kolonienzahl ermittelt und daraus die Keimzahl pro ml Probe bestimmt. Die Anzahl der Impfzyklen, nach denen ein erster Keimbefall zu beobachten ist, ist in Tabelle 2 gezeigt. Diese stellt ein Maß für die Wirksamkeit der Biozide in den jeweiligen Proben dar. Als besonders wirksam erwiesen sich die Formulierungen der Beispiele 33, 34, 35 bzw. 38 auf der Basis der Verbindungen der Beispiele 2, 4, 6 bzw. 16. Beispiel 34 zeigt sogar ohne Zusatz von Pyrithion oder dessen Derivaten eine fungizide Wirkung. Bei einem Vergleich der Beispiele 32 bzw. 42 mit den Beispielen 33 bzw. 36 zeigte sich weiterhin, daß überraschenderweise die Alkanolamide eine bessere Wirksamkeit aufweisen als die von den gleichen Alkanolaminen abgeleiteten Alkanolammoniumsalze.

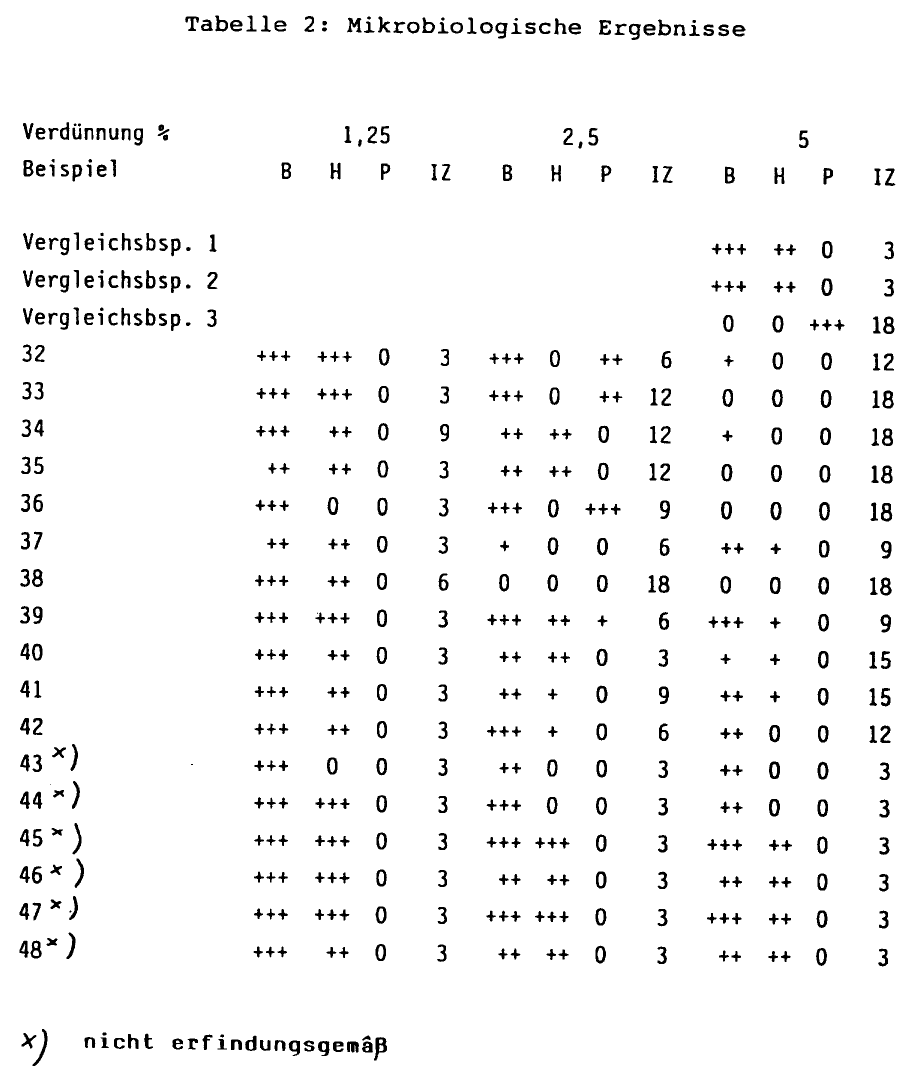

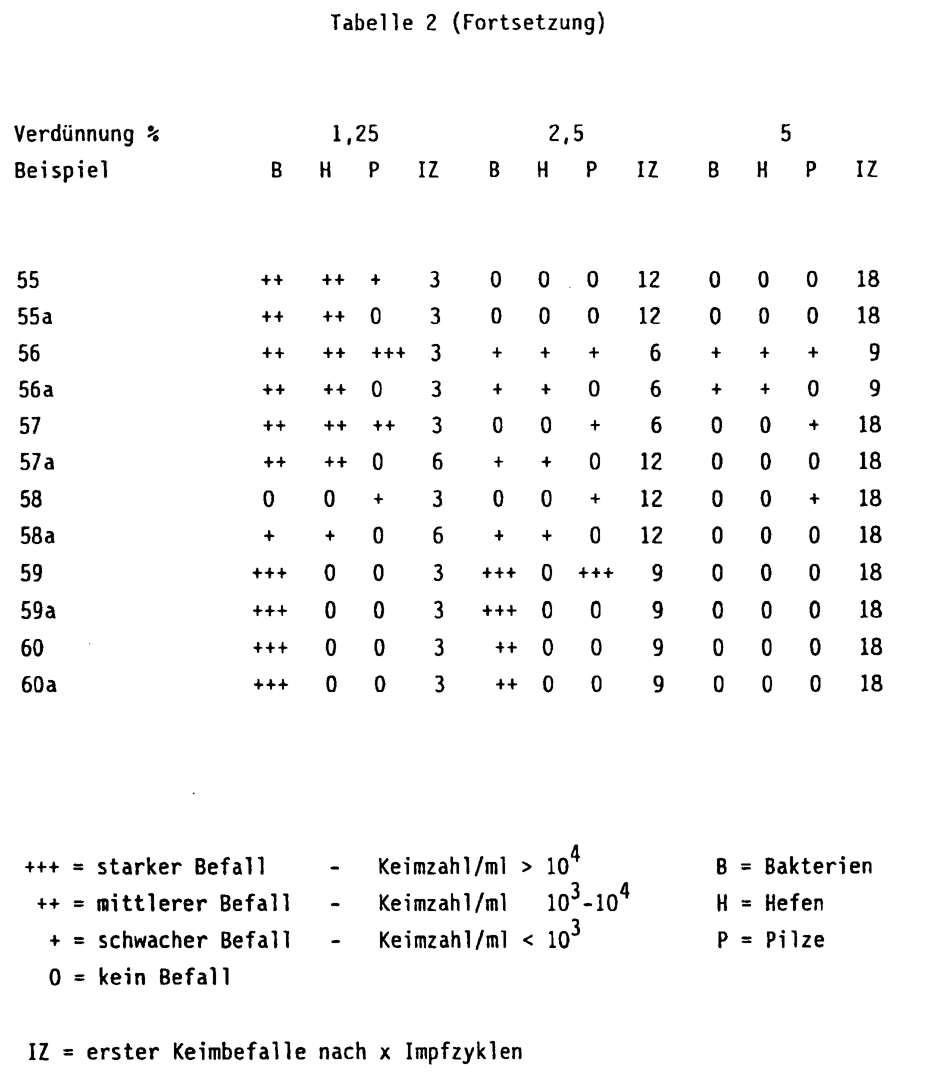

## Patentansprüche

1. Verwendung von 1,3,5-Triazin-2,4,6-tris-alkylaminocarbonsäurederivaten der allgemeinen Formel 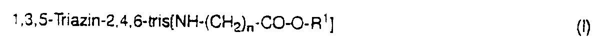 in der
n eine Zahl im Bereich von 4 bis 11 bedeutet und
R¹ eine der folgenden Bedeutungen aufweist:
a) ein Alkaliatom oder ein Ammoniumion, abgeleitet von einem Alkanolamin der allgemeinen Formel 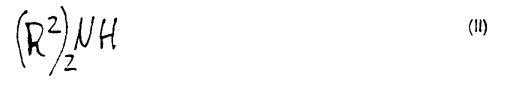 in der
mindestens eine der Gruppen R²
aa) eine Hydroxyalkylgruppe mit 2 bis 4 Konlenstoffatomen,
bb) eine Hydroxyalkyl-oxyalkylengruppe mit jeweils 2 bis 4 Kohlenstoffatomen in dem Hydroxyalkyl- und Oxyalkylenrest, oder
cc) eine Dihydroxyalkylgruppe mit 3 bis 6 Koblenstoffatomen
und, wenn weniger als zwei der Gruppen R² die vorstehende Bedeutung aufweisen, die übrige Gruppe R² eine Alkylgruppe mit 1 bis 6 Kohlenstoffatomen oder Wasserstoff ist, bedeutet,
oder
b) einen Rest eines Alkanolamins der allgemeinen Formel II,
als biozide bzw. biostatische Mittel in wasserhaltigen Systemen.

2. Verwendung nach Anspruch 1, dadurch gekennzeichnet, daß n die Zahl 5 bedeutet.

3. Verwendung nach Anspruch 1 oder 2, dadurch gekennzeichnet, daß die Amine der allgemeinen Formel II primäre Alkanolamine oder Gemische aus primären und sekundären Alkanolaminen mit 2 bis 4 Konlenstoffatomen in den Hydroxyalkylresten sind.

4. Verwendung nach mindestens einem der Ansprüche 1 bis 3 zusammen mit wasserlöslichen, alkalistatilen Fungiziden.

5. Verwendung nach mindestens einem der Ansprüche 1 bis 4, dadurch gekennzeichnet, daß als Fungizid Pyrithion oder Derivate desselben und/oder N-Alkyl-diazeniumdioxidsalze verwendet werden.

6. Verwendung nach mindestens einem der Ansprüche 1 bis 5, dadurch gekennzeichnet, daß die wasserhaltigen Systeme Kühlschmierstoffe sind.

7. Verwendung nach mindestens einem der Anspruche 1 bis 5, dadurch gekennzeicnnet, daß das wasserhaltige System, bezogen auf die Gesamtformulierung, 0,05 bis 0,40 Gew.-% der 1,3,5-Triazin-2,4,6-tris-alkylaminocarbonsäurederivate der allgemeinen Formel I und 0,0001 bis 0,2 Gew.-% Fungizide enthält.

## Claims

1. Use of 1,4,5-triazine-2,4,6-tris-alkylaminocarboxylic acid derivatives of the general formula 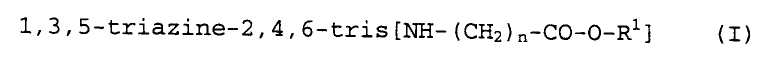 in which
n denotes a number in the range from 4 to 11 and
R¹ has one of the following meanings:
a) an alkali metal atom or an ammonium ion derived from an alkanolamine of the general formula 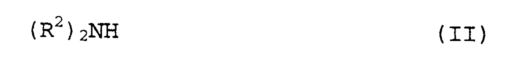 in which
at least one of the groups R² denotes
aa)a hydroxyalkyl group having 2 to 4 carbon atoms,
bb)a hydroxyalkyl-oxyalkylene group having in each case 2 to 4 carbon atoms in the hydroxyalkyl and oxyalkylene radical or
cc)a dihydroxyalkyl group having 3 to 6 carbon atoms
and, if less than two of the groups R² have the above meaning, the other group R² is an alkyl group having 1 to 6 carbon atoms or hydrogen, or
b) a radical of an alkanolamine of the general formula II,
as biocidal and biostatic agents in aqueous systems.

2. Use according to Claim 1, characterized in that n denotes the number 5.

3. Use according to Claim 1 or 2, characterized in that the amines of the general formula II are primary alkanolamines or mixtures of primary and secondary alkanolamines having 2 to 4 carbon atoms in the hydroxyalkyl radicals.

4. Use according to at least one of Claims 1 to 3 together with water-soluble fungicides which are stable to alkali.

5. Use according to at least one of Claims 1 to 4, characterized in that pyrithione or derivatives thereof and/or N-alkyl-diazenium dioxide salts are used as the fungicide.

6. Use according to at least one of Claims 1 to 5, characterized in that the aqueous systems are cooling lubricants.

7. Use according to at least one of Claims 1 to 6, characterized in that the aqueous system, based on the total formulation, contains 0.05 to 0.40% by weight of the 1,3,5-triazine-2,4,6-tris-alkylaminocarboxylic acid derivatives of the general formula I and 0.0001 to 0.2% by weight of fungicides.

## Revendications

1. Utilisation de dérivés des acides 1,3,5-triazine-2,4,6-tris-alkylaminocarboxyliques de formule générale 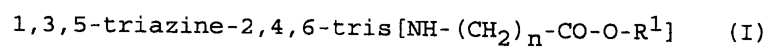 dans laquelle
n désigne un nombre dans le domaine de 4 à 11 et
R¹ présente une des significations suivantes :
a) un atome alcalin ou un ion ammonium, dérivé d'une alcanolamine de formule générale 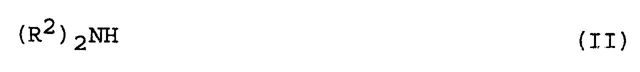 dans laquelle
au moins un des groupes R² désigne
aa) un groupement hydroxyalkyle ayant 2 à 4 atomes de carbone,
bb) un groupe hydroxyalkyl-oxyalkylène ayant à chaque fois de 2 à 4 atomes de carbone dans le résidu hydroxyalkyle et oxyalkylène,
ou
cc) un groupement dihydroxyalkyle ayant 3 à 6 atomes de carbone,
et si moins de deux des groupements R² présentent la signification précédente, l'autre groupement R² est un groupement alkyle ayant 1 à 6 atomes de carbone ou de l'hydrogène,
ou
b) un résidu d'une alcanolamine de formule générale II,
en tant qu'agent biocide, respectivement en tant qu'agent biostatique dans des systèmes contenant de l'eau.

2. Utilisation selon la revendication 1, caractérisée en ce que n représente le nombre 5.

3. Utilisation selon la revendication 1 ou 2, caractérisée en ce que les amines de la formule générale II sont des alcanolamines primaires ou des mélanges d'alcanolamines primaires et secondaires ayant de 2 à 4 atomes de carbone dans les résidus hydroxyalkyle.

4. Utilisation selon au moins l'une quelconque des revendications 1 à 3, conjointement à des fongicides hydrosolubles, stables vis-à-vis des alcalins.

5. Utilisation selon au moins l'une quelconque des revendications 1 à 4, caractérisée en ce que l'on utilise en tant que fongicide le pyrithion ou des dérivés de ce dernier et/ou des sels de dioxyde de N-alkyl-diazénium.

6. Utilisation selon au moins l'une quelconque des revendications 1 à 5, caractérisée en ce que les systèmes contenant de l'eau sont des réfrigérants lubrifiants.

7. Utilisation selon au moins l'une quelconque des revendications 1 à 6, caractérisée en ce que le système contenant de l'eau contient, par rapport à la formulation dans son ensemble, de 0,05 à 0,40 % en poids du dérivé d'acide 1,3,5-triazine-2,4,6-tris-alkylamino-carboxylique de formule générale I et de 0,0001 à 0,2 % en poids de fongicides.
